# EUROPÄISCHE PATENTSCHRIFT

(11) **EP 0 767 165 B1**
(45) Veröffentlichungstag und Bekanntmachung des Hinweises auf die Patenterteilung: **08.12.1999**
(21) Anmeldenummer: 96115627.0
(22) Anmeldetag: 30.09.1996
(51) Int. Cl.: C07C 253/28, B01J 23/90, B01J 23/18

(54) **Verfahren zur Herstellung von aromatischen oder heteroaromatischen Nitrilen**
Process for the preparation of aromatic or heteroaromatic nitriles
Procédé de préparation de nitriles aromatiques ou hétéroaromatiques

(30) Priorität: 07.10.1995 DE 19537446
(43) Veröffentlichungstag der Anmeldung: 09.04.1997
(73) Patentinhaber: BASF Aktiengesellschaft, 67063 Ludwigshafen (DE)
(72) Erfinder: Karrer, Lothar, Dr., 64319 Pfungstadt (DE); Pape, Frank-Friedrich, Dr., 67259 Kleinniedesheim (DE); Kneuper, Heinz-Josef, Dr., 68163 Mannheim (DE); Hüllmann, Michael, Dr., 64625 Bensheim (DE)

(56) Entgegenhaltungen:
- EP-A- 0 043 684
- EP-A- 0 153 077
- EP-A- 0 222 249
- US-A- 4 107 085
- US-A- 4 784 979
- PATENT ABSTRACTS OF JAPAN vol. 6, no. 227 (C-134), 12.November 1982 & JP-A-57 130546 (MITSUI TOATSU KAGAKU KK), 13.August 1982,

## Beschreibung

Die vorliegende Erfindung betrifft ein Verfahren zur Herstellung von aromatischen oder heteroaromatischen Nitrilen aus aromatischen oder heteroaromatischen Kohlenwasserstoffen mit Ammoniak und Sauerstoff oder sauerstoffenthaltenden Gasen bei erhöhten Temperaturen in der Gasphase an neuen Trägerkatalysatoren, die Vanadiumoxid enthalten, und aus zwei bis dreißig Kornfraktionen bestehen, deren mittlerer Durchmesser sich um 10 bis 80 % unterscheiden mit einer bestimmten Schüttdichte des Trägers.

Aus der EP-A-222 249 ist ein Verfahren zur Herstellung von aromatischen Nitrilen aus alkylsubstituierten aromatischen Kohlenwasserstoffen durch katalytische Oxidation mit Ammoniak und Sauerstoff oder sauerstoffenthaltenden Gasen bei erhöhter Temperatur in der Dampfphase in Gegenwart eines Katalysators, der 2 bis 10 Gew.-% Vanadiumpentoxid, 1 bis 10 Gew.-% Antimontrioxid, 0,02 bis 2 Gew.-% Alkalioxid und 0,01 bis 1 Gew.-% Erdalkalimetalloxid auf Aluminiumoxid enthält, bekannt. Diese Katalysatoren bestehen aus nur einer Kornfraktion.

Aus der DE-A-28 10 856 ist ein Verfahren zur Herstellung von o-Aminobenzonitril aus o-Toluidin durch katalytische Oxidation mit Ammoniak und Sauerstoff bei erhöhter Temperatur in der Dampfphase in Gegenwart eines Katalysators, der 1 bis 9 Gew.-% Vanadiumoxid, 5 bis 12 Gew.-% Antimonoxid, 3 bis 9 Gew.-% Bismutoxid, 0,5 bis 4 Gew.-% Phosphoroxid auf einem Träger enthält, bekannt. Auch diese Katalysatoren bestehen nur aus einer Kornfraktion.

Bei den bislang bekannten Verfahren waren die Sauerstoffaufnahme des Katalysators und die Ausbeuten verbesserungsbedürftig.

Der vorliegenden Erfindung lag daher die Aufgabe zugrunde, den zuvor genannten Nachteilen abzuhelfen.

Demgemäß wurde ein neues und verbessertes Verfahren zur Herstellung von aromatischen oder heteroaromatischen Nitrilen der allgemeinen Formel I 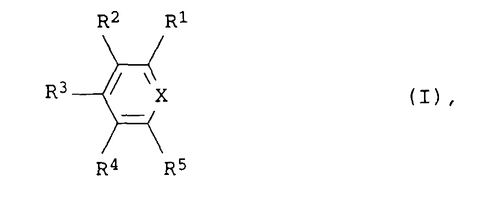 in der
- X: Stickstoff oder C-R⁶ und
- R¹,R²,R³,R⁴,R⁵,R⁶: Wasserstoff, C₁- bis C₈-Alkyl, Halogen, Trifluormethyl, Nitro, Amino, Cyano, C₁- bis C₇-Cyanoalkyl, C₁- bis C₈-Aminoalkyl oder Hydroxy, mit der Maßgabe, daß mindestens einer der Substituenten für Cyano oder C₁- bis C₇-Cyanoalkyl steht,
bedeuten, aus aromatischen oder heteroaromatischen Kohlenwasserstoffen der allgemeinen Formel II 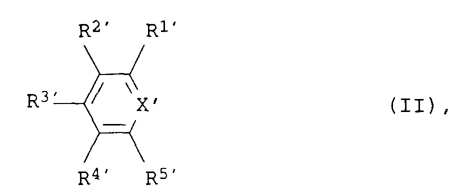 in der
- X': Stickstoff oder C-R^{6'} und
- R^{1'},R^{2'},R^{3'},R^{4'},R^{5'},R^{6'}: Wasserstoff, C₁- bis C₈-Alkyl, Halogen, Trifluormethyl, Nitro, Amino, C₁- bis C₈-Aminoalkyl oder Hydroxy, mit der Maßgabe, daß mindestens einer der Substituenten für C₁- bis C₈-Alkyl steht,
bedeuten, mit Ammoniak und Sauerstoff oder sauerstoffenthaltenden Gasen bei Temperaturen von 200 bis 600°C und einem Druck von 0,1 bis 5 bar in der Gasphase an Trägerkatalysatoren, die 0,5 bis 20 Gew.-% Vanadiumoxid enthalten, gefunden, welches dadurch gekennzeichnet ist, daß die Trägerkatalysatoren aus zwei bis dreißig Kornfraktionen bestehen, deren mittlerer Durchmesser sich um 10 bis 80 % unterscheiden und der Träger eine Schüttdichte von 0,6 bis 1,2 kg/l aufweist sowie neue Trägerkatalysatoren.

Das erfindungsgemäße Verfahren läßt sich wie folgt durchführen:

Ein Gemisch aus einem aromatischen oder heteroaromatischen Kohlenwasserstoff, Ammoniak und Sauerstoff oder einem sauerstoffenthaltenden Gas kann in der Gasphase bei Temperaturen von 200 bis 600°C, bevorzugt 300 bis 550°C, besonders bevorzugt 350 bis 500°C und einem Druck von 0,1 bis 5 bar, bevorzugt 0,3 bis 2 bar, besonders bevorzugt 0,5 bis 1,5 bar, insbesondere bei Normaldruck (Atmosphärendruck) in Gegenwart eines Trägerkatalysators, der aus zwei bis dreißig, bevorzugt zwei bis zehn, besonders bevorzugt zwei bis fünf, also 2, 3, 4 oder 5, insbesondere zwei oder drei Kornfraktionen besteht, deren mittlerer Durchmesser sich um 10 bis 80 %, bevorzugt 10 bis 70 %, besonders bevorzugt 30 bis 60 % unterscheiden und der Träger eine Schüttdichte von 0,6 bis 1,2 kg/l, bevorzugt 0,6 bis 1,0 kg/l, besonders bevorzugt 0,6 bis 0,9 kg/l aufweist, im Festbett, bevorzugt im Wirbelbett umgesetzt werden.

Vorzugsweise nimmt man die Ausgangsverbindungen in einem Gasstrom aus Ammoniak und einem Sauerstoff enthaltenden Gas wie Luft auf, wobei ihre Konzentration zweckmäßigerweise auf 0,1 bis 25 Vol.-%, vorzugsweise auf 0,1 bis 10 Vol.-%, eingestellt wird.

Als Trägerkatalysatoren eignen sich solche, mit Trägern aus Aluminiumoxid, Siliciumdioxid, Titandioxid, Zirkoniumdioxid, Siliciumcarbid, Magnesiumoxid oder deren Gemische, bevorzugt Aluminiumoxid, Siliciumdioxid, Titandioxid, Zirkoniumdioxid oder deren Gemische, besonders bevorzugt Aluminiumoxid, Siliciumdioxid oder deren Gemische und 0,5 bis 20 Gew.-%, bevorzugt 1 bis 10 Gew.-%, besonders bevorzugt 3 bis 8 Gew.-% Vanadiumoxid und 0 bis 20 Gew.-%, bevorzugt 0,5 bis 20 Gew.-%, besonders bevorzugt 1 bis 10 Gew.-%, insbesondere 2 bis 8 Gew.-% Antimonoxid und 0 bis 4 Gew.-%, bevorzugt 0,01 bis 4 Gew.-%, besonders bevorzugt 0,05 bis 3 Gew.-% Cäsiumoxid, Rubidiumoxid oder deren Gemische und 0 bis 10 Gew.-%, bevorzugt 0 bis 5 Gew.-%, besonders bevorzugt 0 bis 3 Gew.-% eines oder mehrerer Oxide aus der Gruppe Wolfram, Molybdän, Titan, Eisen, Cobalt, Nickel, Mangan, Kupfer oder ein Alkalimetall oder Erdalkalimetall, wobei der Träger 50 bis 99,9 Gew.-% der Gesamtkatalysatormasse ausmacht.

Die Trägerkatalysatoren lassen sich durch gleichzeitiges oder nacheinander Tränken oder Imprägnieren des Trägers, mit einer beliebigen Lösungen, vorzugsweise mit einer wäßrigen Lösungen bzw. einer Suspension der einer oder mehrerer Verbindungen der aktiven Katalysatorbestandteile wie Vanadium und gegebenenfalls Antimon, Cäsium, Rubidium, Wolfram, Molybdän, Titan, Eisen, Cobalt, Nickel, Mangan, Kupfer, Alkalimetall oder Erdalkalimetall, Trocknung und Calcinierung unter oxidierenden Bedingungen bei Temperaturen von 400 bis 800°C, bevorzugt 450 bis 750°C herstellen. Vorzugsweise setzt man die Imprägnierlösung bzw. -suspension nicht in größerer Menge ein, als vom Trägermaterial aufgenommen werden kann. Man kann die Imprägnierung nach jeweiliger Zwischentrocknung auch in mehreren Schritten vornehmen.

Als Tränk- oder Imprägnierlösungen setzt man in der Regel die aktiven Komponenten vorzugsweise in Form von wäßrigen Lösungen ihrer Salze ein, und zwar insbesondere von Salzen organischer Säuren, die sich bei der oxidativen Calcinierung rückstandsfrei zersetzen lassen. Bevorzugt werden hierbei die Oxalate, besonders im Fall des Vanadiums, und die Tartrate, besonders im Fall des Antimons und Wolframs, wobei die Tartrate auch in Form gemischter Salze, z.B. mit Ammoniumionen vorliegen können. Zur Herstellung solcher Lösungen kann man die Metalloxide in den Säuren auflösen.

Die Träger unterschiedlicher mittlerer Korngrößen können vor oder bevorzugt nach den Tränk- oder Imprägniervorgängen vereinigt und bevorzugt intensiv vermischt werden.

Die Substituenten R¹, R², R³, R⁴, R⁵, R⁶, R^{1'}, R^{2'}, R^{3'}, R^{4'}, R^{5'}, R^{6'} und das Zwischenglied X und X' haben folgende Bedeutungen:
X, X'
   - Stickstoff oder C-R⁶, bevorzugt C-R⁶,
R¹,R²,R³,R⁴,R⁵,R⁶,R^{1'},R^{2'},R^{3'},R^{4'},R^{5'},R^{6'}
   - Wasserstoff,
   - C₁- bis C₈-Alkyl, bevorzugt C₁- bis C₄-Alkyl, besonders bevorzugt Methyl, Ethyl, n-Propyl und iso-Propyl,
   - Halogen wie Fluor, Chlor, Brom oder Jod, bevorzugt Fluor, Chlor oder Brom, besonders bevorzugt Chlor oder Brom, insbesondere Chlor,
   - Trifluormethyl,
   - Nitro,
   - Amino,
   - C₁- bis C₈-Aminoalkyl, bevorzugt C₁- bis C₄-Aminoalkyl, besonders bevorzugt Aminomethyl, 1-Aminoethyl und 2-Aminoethyl,
   - Hydroxy,
R¹,R²,R³,R⁴,R⁵,R⁶ zusätzlich
   - Cyano,
   - C₁- bis C₇-Cyanoalkyl, bevorzugt C₁- bis C₃-Cyanoalkyl, besonders bevorzugt Cyanomethyl, 1-Cyanoethyl und 2-Cyanoethyl,
   - mit der Maßgabe, daß mindestens einer, also 1, 2, 3, 4, 5 oder 6, bevorzugt 1, 2 oder 3, besonders bevorzugt 1 oder 2, insbesondere einer der Substituenten für Cyano oder C₁- bis C₇-Cyanoalkyl steht.

Besondere technische Bedeutung hat die Ammonoxidation für die Herstellung von Phthalodinitril aus o-Xylol, von Isophthalodinitril aus m-Xylol, von Terephthalodinitril aus p-Xylol, von Benzonitril aus Toluol und von Nicotinsäurenitril aus β-Picolin.

Im Falle der Xylole verläuft die Ammonoxidation der ersten Methylgruppe schneller als die der zweiten, so daß sich auch leicht partielle Ammonoxidationsprodukte gewinnen lassen, z.B. p-Methylbenzonitril aus p-Xylol, o-Methylbenzonitril aus o-Xylol und gegebenenfalls Benzonitril als Nebenprodukt.

### Beispiel

### Katalysator A

1000 g kugelförmiges Aluminiumoxid (Puralox® SCC a 150/120 der Firma Condea-Chemie), welches eine Schüttdichte von 0,8 kg/l hatte, wurde in einem Mischer mit 930,4 g einer wäßrigen Lösung imprägniert, die aus 351,4 g destilliertem Wasser, 150 g 25 %iger Ammoniak-Lösung, 65,5 g Antimon-(III)-oxid, 56,2 g Vanadiumsäure (90 Gew.-% Vanadium-(V)-oxid), 6 g Kaliumnitrat, 150 g Weinsäure und 152,3 g Oxalsäuredihydrat hergestellt worden war. Der feuchte Katalysatorvorläufer wurde bei 600°C in Anwesenheit von Luft über den Zeitraum von 10 h getempert.

### Katalysator B

1000 g kugelförmiges Aluminiumoxid (Puralox® SCC a 80/120 Firma Condea-Chemie) wurden wie bei Katalysator A beschrieben mit der Aktivmassenlösung versetzt. Der feuchte Katalysatorvorläufer wurde bei 600°C in Anwesenheit von Luft über den Zeitraum von 10 h getempert.

Die beiden Kornfraktionen A und B unterscheiden sich nach Siebanalyse im mittleren Durchmesser um 45 % relativ.

### Beispiele 1 und 2 und Vergleichsbeispiele A und B

### Ammonoxidation von o-Xylol

In einem Wirbelschichtreaktor von 6 cm Durchmesser und 120 cm Höhe wurde bei 470°C ein Gasstrom bestehend aus 3 Vol.-% o-Xylol (d.h. 120 g/h), 12 Vol.-% Sauerstoff und 85 Vol.-% Ammoniak über 600 g Katalysator geleitet. Es wurden vier verschiedene Katalysatoren eingesetzt.

Beispiel 1: Gemisch aus 550 g Katalysator A + 50 g Katalysator B Beispiel 2: Gemisch aus 500 g Katalysator A + 100 g Katalysator B Vergleichsbeispiel A: Reinfraktion von 600 g Katalysator A Vergleichsbeispiel B: Reinfraktion von 600 g Katalysator B

Die Ergebnisse sind in der folgenden Tabelle zusammengestellt:

**Tabelle**

| | Vergleichs-beispiel A | Vergleichs-beispiel B | Beispiel 1 | Beispiel 2 |
|---|---|---|---|---|
| Menge Katalysator [g] | 600 | 600 | 600 | 600 |
| Katalysator A [Gew.-%] | 100 | 0 | 95 | 90 |
| Katalysator B [Gew.-%) | 0 | 100 | 5 | 10 |
| Temperatur [°C] | 470 | 470 | 470 | 470 |
| Menge o-Xylol [g/h] | 120 | 120 | 120 | 120 |
| Umsatz o-Xylol [%] | 93,5 | 93,7 | 94,5 | 94 |
| Phthalodinitril-Ausbeute [Mol.-%] | 61,5 | 61,7 | 64,2 | 64,1 |
| g Phthalodinitril/h/100g Kat. | 89,1 | 89,4 | 93 | 92,9 |

## Patentansprüche

1. Verfahren zur Herstellung von aromatischen oder heteroaromatischen Nitrilen der allgemeinen Formel I 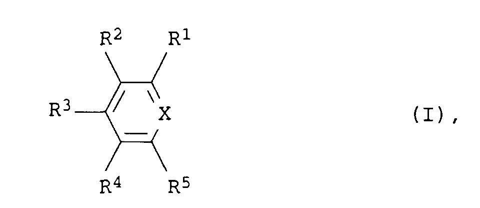 in der
X Stickstoff oder C-R⁶ und
R¹,R²,R³,R⁴,R⁵,R⁶ Wasserstoff, C₁- bis C₈-Alkyl, Halogen, Trifluormethyl, Nitro, Amino, Cyano, C₁-bis C₇-Cyanoalkyl, C₁- bis C₈-Aminoalkyl oder Hydroxy, mit der Maßgabe, daß mindestens einer der Substituenten für Cyano oder C₁- bis C₇-Cyanoalkyl steht,
bedeuten, aus aromatischen oder heteroaromatischen Kohlenwasserstoffen der allgemeinen Formel II 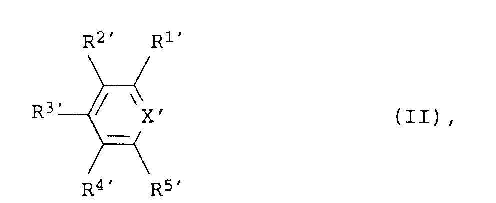 in der
X' Stickstoff oder C-R^{6'} und
R^{1'} ,R^{2'} ,R^{3'} ,R^{4'} ,R^{5'} ,R^{6'} Wasserstoff, C1- bis C₈-Alkyl, Halogen, Trifluormethyl, Nitro, Amino, C₁- bis C₈-Aminoalkyl oder Hydroxy, mit der Maßgabe, daß mindestens einer der Substituenten für C₁- bis C₈-Alkyl steht,
bedeuten, mit Ammoniak und Sauerstoff oder sauerstoffenthaltenden Gasen bei Temperaturen von 200 bis 600°C und einem Druck von 0,1 bis 5 bar in der Gasphase an Trägerkatalysatoren, die 0,5 bis 20 Gew.-% Vanadiumoxid enthalten, dadurch gekennzeichnet, daß die Trägerkatalysatoren aus zwei bis dreißig Kornfraktionen bestehen, deren mittlerer Durchmesser sich um 10 bis 80 % unterscheiden und der Träger eine Schüttdichte von 0,6 bis 1,2 kg/l aufweist.

2. Verfahren zur aromatischen oder heteroaromatischen Nitrilen nach Anspruch 1, dadurch gekennzeichnet, daß man die Umsetzung in der Wirbelschicht durchführt.

3. Verfahren zur aromatischen oder heteroaromatischen Nitrilen nach den Ansprüchen 1 bis 2, dadurch gekennzeichnet, daß der Träger aus Aluminiumoxid, Siliciumdioxid, Titandioxid, Zirkoniumdioxid oder deren Gemischen besteht.

4. Verfahren zur aromatischen oder heteroaromatischen Nitrilen nach den Ansprüchen 1 bis 3, dadurch gekennzeichnet, daß der Trägerkatalysator 0,5 bis 20 Gew.-% Vanadiumoxid und 0,5 bis 20 Gew.-% Antimonoxid enthält.

5. Verfahren zur aromatischen oder heteroaromatischen Nitrilen nach den Ansprüchen 1 bis 4, dadurch gekennzeichnet, daß der Trägerkatalysator 0,5 bis 20 Gew.-% Vanadiumoxid und 0,5 bis 20 Gew.-% Antimonoxid und 0,01 bis 4 Gew.-% Cäsiumoxid, Rubidiumoxid oder deren Gemische enthält.

6. Trägerkatalysatoren aus zwei bis dreißig Kornfraktionen, deren mittlerer Durchmesser sich um 10 bis 80 % unterscheiden und deren Träger eine Schüttdichte von 0,6 bis 1,2 kg/l aufweisen und die 0,5 bis 20 Gew.-% Vanadiumoxid, 0 bis 20 Gew.-% Antimonoxid, 0 bis 4 Gew.-% Cäsiumoxid, Rubidiumoxid 0 bis 10 Gew.-% eines oder mehrerer Oxide aus der Gruppe Wolfram, Molybdän, Titan, Eisen, Cobalt, Nickel, Mangan, Kalium, Kupfer oder ein Erdalkalimetall, wobei der Träger 50 bis 99,9 Gew.-% der Gesamtkatalysatormasse ausmacht.

7. Trägerkatalysatoren nach Anspruch 6, dadurch gekennzeichnet, daß man als Träger Aluminiumoxid, Siliciumdioxid, Titandioxid, Zirkoniumdioxid oder deren Gemische einsetzt.

8. Trägerkatalysatoren nach den Ansprüchen 6 und 7, dadurch gekennzeichnet, daß man als Träger Aluminiumoxid, Siliciumdioxid oder deren Gemische einsetzt.

9. Verfahren zur Herstellung der Trägerkatalysatoren nach den Ansprüchen 6 bis 8, dadurch gekennzeichnet, daß man Träger mit unterschiedlichen mittleren Korndurchmessern mit der Lösung oder Suspension aus 0,5 bis 20 Gew.-% Vanadiumoxid, 0 bis 20 Gew.-% Antimonoxid, 0 bis 4 Gew.-% Cäsiumoxid, Rubidiumoxid 0 bis 10 Gew.-% eines oder mehrerer Oxide aus der Gruppe Wolfram, Molybdän, Titan, Eisen, Cobalt, Nickel, Mangan, Kupfer oder ein Alkalimetall oder Erdalkalimetall, wobei der Träger 50 bis 99,9 Gew.-% der Gesamtkatalysatormasse ausmacht, gleichzeitig oder nacheinander tränkt oder imprägniert, trocknet und unter oxidierenden Bedingungen bei Temperaturen von 400 bis 800°C calciniert.

10. Verfahren zur Herstellung der Trägerkatalysatoren nach Anspruch 9, dadurch gekennzeichnet, daß man Vanadium als Oxalat und Antimon als Tartrat einsetzt.

## Claims

1. A process for the preparation of aromatic or heteroaromatic nitriles of the formula I 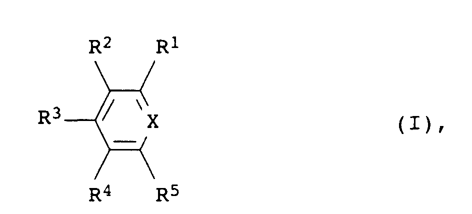 where
X is nitrogen or C-R⁶ and
R¹, R², R³, R⁴, R⁵ and R⁶ are each hydrogen, C₁-C₈-alkyl, halogen, trifluoromethyl, nitro, amino, cyano, C₁-C₇-cyanoalkyl, C₁-C₈-aminoalkyl or hydroxyl, with the proviso that at least one of the substituents is cyano or C₁-C₇-cyanoalkyl,
from aromatic or heteroaromatic hydrocarbons of the formula II 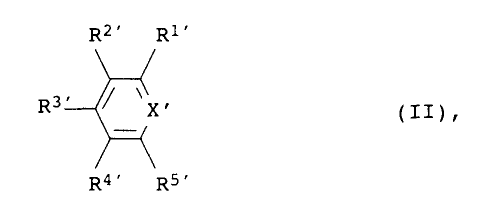 where
X' is nitrogen or C-R^{6'} and
R^{1'}, R^{2'}, R^{3'}, R^{4'}, R^{5'} and R^{6'} are each hydrogen, C₁-C₈-alkyl, halogen, trifluoromethyl, nitro, amino, C₁-C₈-aminoalkyl or hydroxyl, with the proviso that at least one of the substituents is C₁-C₈-alkyl,
with ammonia and oxygen or oxygen-containing gases at from 200 to 600°C and from 0.1 to 5 bar in the gas phase over supported catalysts which contain from 0.5 to 20% by weight of vanadium oxide, wherein the supported catalysts consist of from 2 to 30 particle fractions whose mean diameters differ by from 10 to 80% and the carrier has a bulk density of from 0.6 to 1.2 kg/l.

2. A process for the preparation of aromatic or heteroaromatic nitriles as claimed in claim 1, wherein the reaction is carried out in a fluidized bed.

3. A process for the preparation of aromatic or heteroaromatic nitriles as claimed in claims 1 and 2, wherein the carrier consists of alumina, silica, titanium dioxide, zirconium dioxide or a mixture thereof.

4. A process for the preparation of aromatic or heteroaromatic nitriles as claimed in any of claims 1 to 3, wherein the supported catalyst contains from 0.5 to 20% by weight of vanadium oxide and from 0.5 to 20% by weight of antimony oxide.

5. A process for the preparation of aromatic or heteroaromatic nitriles as claimed in any of claims 1 to 4, wherein the supported catalyst contains from 0.5 to 20% by weight of vanadium oxide, from 0.5 to 20% by weight of antimony oxide and from 0.01 to 4% by weight of cesium oxide, rubidium oxide or a mixture thereof.

6. A supported catalyst comprising from 2 to 30 particle fractions whose mean diameters differ by from 10 to 80% and whose carrier has a bulk density of from 0.6 to 1.2 kg/l and which contains from 0.5 to 20% by weight of vanadium oxide, from 0 to 20% by weight of antimony oxide, from 0 to 4% by weight of cesium oxide or rubidium oxide and from 0 to 10% by weight of one or more oxides selected from the group consisting of tungsten, molybdenum, titanium, iron, cobalt, nickel, manganese, potassium, copper or an alkaline earth metal, the carrier accounting for from 50 to 99.9% by weight of the total catalyst mass.

7. A supported catalyst as claimed in claim 6, wherein alumina, silica, titanium dioxide, zirconium dioxide or a mixture thereof is used as the carrier.

8. A supported catalyst as claimed in claims 6 and 7, wherein alumina, silica or a mixture thereof is used as the carrier.

9. A process for the preparation of the supported catalysts as claimed in any of claims 6 to 8, wherein a carrier having different mean particle diameters is impregnated simultaneously or in succession with a solution or suspension of from 0.5 to 20% by weight of vanadium oxide, from 0 to 20% by weight of antimony oxide, from 0 to 4% by weight of cesium oxide or rubidium oxide and from 0 to 10% by weight of one or more oxides selected from the group consisting of tungsten, molybdenum, titanium, iron, cobalt, nickel, manganese, copper or an alkali metal or alkaline earth metal, the carrier accounting for from 50 to 99.9% by weight of the total catalyst mass, is dried and is calcined under oxidizing conditions at from 400 to 800°C.

10. A process for the preparation of the supported catalysts as claimed in claim 9, wherein vanadium in the form of the oxalate and antimony in the form of the tartrate are used.

## Revendications

1. Procédé de préparation de nitriles aromatiques ou hétéroaromatiques de formule générale I 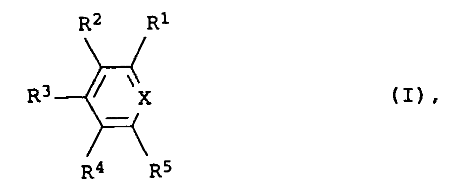 dans laquelle
X représente un atome d'azote ou C-R⁶ et
R¹, R², R³, R⁴, R⁵, R⁶ représentent un atome d'hydrogène, un groupement alkyle en C₁-C₈, un atome d'halogène, un groupement trifluorométhyle, nitro, amino, cyano, cyanoalkyle en C₁-C₇, aminoalkyle en C₁-C₈ ou hydroxy, étant spécifié que l'un au moins des substituants est mis pour un groupement cyano ou cyanoalkyle en C₁-C₇,
à partir d'hydrocarbures aromatiques ou hétéroaromatiques de formule générale II 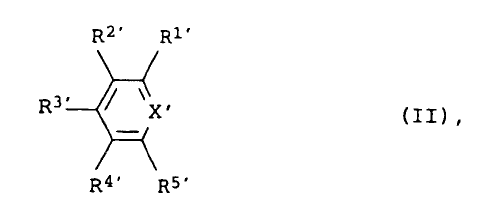 dans laquelle
X' représente un atome d'azote ou C-R^{6'} et
R^{1'}, R^{2'}, R^{3'}, R^{4'}, R^{5'}, R^{6'} représentent un atome d'hydrogène, un groupement alkyle en C₁-C₈, un atome d'halogène, un groupement trifluorométhyle, nitro, amino, aminoalkyle en C₁-C₈ ou hydroxy, étant spécifié que l'un au moins des substituants est mis pour un groupement alkyle en C₁-C₈,
avec de l'ammoniac et de l'oxygène ou des gaz contenant de l'oxygène, à des températures de 200-600°C et sous une pression de 0,1-5 bar en phase gazeuse sur des catalyseurs sur supports contenant 0,5-20% en poids d'oxyde de vanadium, caractérisé en ce que les catalyseurs sur supports sont constitués de deux à trente fractions granulométriques, dont les diamètres moyens se différencient de 10 à 80% et en ce que le support présente une densité apparente de 0,6-1,2 kg/l.

2. Procédé de préparation de nitriles aromatiques ou hétéroaromatiques selon la revendication 1, caractérisé en ce que l'on mène la réaction en lit fluidisé.

3. Procédé de préparation de nitriles aromatiques ou hétéroaromatiques selon la revendication 1 ou 2, caractérisé en ce que le support est constitué d'oxyde d'aluminium, de dioxyde de silicium, de dioxyde de titane, de dioxyde de zirconium ou de leurs mélanges.

4. Procédé de préparation de nitriles aromatiques ou hétéroaromatiques selon l'une quelconque des revendications 1 à 3, caractérisé en ce que le catalyseur sur support contient 0,5-20% en poids d'oxyde de vanadium et 0,5-20% en poids d'oxyde d'antimoine.

5. Procédé de préparation de nitriles aromatiques ou hétéroaromatiques selon l'une quelconque des revendications 1 à 4, caractérisé en ce que le catalyseur sur support contient 0,5-20% en poids d'oxyde de vanadium et 0,5-20% en poids d'oxyde d'antimoine et 0,01-4% en poids d'oxyde de césium, d'oxyde de rubidium ou de leurs mélanges.

6. Catalyseurs sur support formés de deux à trente fractions granulométriques, dont les diamètres moyens se différencient de 10 à 80% et dont le support présente une densité apparente de 0,6-1,2 kg/l, et contenant 0,5-20% en poids d'oxyde de vanadium et 0-20% en poids d'oxyde d'antimoine et 0-4% en poids d'oxyde de césium, d'oxyde de rubidium, 0-10% en poids d'un ou plusieurs oxydes du groupe formé par le tungstène, le molybdène, le titane, le fer, le cobalt, le nickel, le manganèse, le potassium, le cuivre ou un métal alcalinoterreux, le support représentant 50-99,9% en poids de la masse totale du catalyseur.

7. Catalyseur sur support selon la revendication 6, caractérisé en ce que l'on utilise, en tant que support, de l'oxyde d'aluminium, du dioxyde de silicium, du dioxyde de titane, du dioxyde de zirconium ou leurs mélanges.

8. Catalyseur sur support selon la revendication 6 ou 7, caractérisé en ce que l'on utilise, en tant que support, de l'oxyde d'aluminium, du dioxyde de silicium ou leurs mélanges.

9. Procédé de préparation du catalyseur sur support selon l'une quelconque des revendications 6 à 8, caractérisé en ce que l'on immerge ou que l'on imprègne, en même temps ou l'un après l'autre, un support ayant des diamètres moyens de particules différents avec une solution ou une suspension à base de 0,5-20% en poids d'oxyde de vanadium et 0-20% en poids d'oxyde d'antimoine, 0-4% en poids d'oxyde de césium, d'oxyde de rubidium, 0-10% en poids d'un ou plusieurs oxydes du groupe formé par le tungstène, le molybdène, le titane, le fer, le cobalt, le nickel, le manganèse, le cuivre ou un métal alcalinoterreux, le support représentant 50-99,9% en poids de la masse totale du catalyseur, on le sèche et on le calcine dans des conditions oxydantes à des températures de 400-800°C.

10. Procédé de préparation du catalyseur sur support selon la revendication 9, caractérisé en ce que l'on utilise du vanadium sous forme d'oxalate et de l'antimoine sous forme de tartrate.
